# EUROPEAN PATENT APPLICATION

(11) **EP 4 559 897 A1**
(43) Date of publication of application: **28.05.2025**
(21) Application number: 23872503.0
(22) Date of filing: 28.09.2023
(51) Int. Cl.: C07C 309/65, C07C 49/813, C07D 307/46, C07D 333/22

(54) **ASYMMETRIC ALLYL FLUORIDE COMPOUND AND METHOD FOR PRODUCING SAME**

(30) Priority: 30.09.2022 JP 2022158768
(71) Applicant: SynCrest Inc., Fujisawa-shi, Kanagawa 251-8555 (JP); University of The Ryukyus, Nakagami-gun, Okinawa 903-0213 (JP)
(72) Inventor: XU, Pengyu, Fujisawa-shi, Kanagawa 251-8555 (JP); YONEYAMA, Shin, Fujisawa-shi, Kanagawa 251-8555 (JP); ARIMITSU, Satoru, Nakagami-gun, Okinawa 903-0213 (JP); TOMON, Daiki, Nakagami-gun, Okinawa 903-0213 (JP)
(74) Representative: Meissner Bolte Partnerschaft mbB
(86) International application number: PCT/JP2023/035346
(87) International publication number: WO 2024/071281

(57) **Abstract**

An object of the present invention is to provide a novel asymmetric fluorinated allylic compound with high optical purity that can be derived into various physiologically active medium molecules, and a production method for the asymmetric fluorinated allylic compound. Provided is an asymmetric fluorinated allyl compound.

## Description

### Technical Field

The present invention relates to an asymmetric fluorinated allylic compound and a production method for the asymmetric fluorinated allylic compound.

### Background Art

NPL 1 discloses a method for synthesizing an allyl compound having a fluorine atom at the α-position.

### Citation List

### Non-patent Literature

NPL 1: Advances in Nucleophilic Allylic Fluorination. ACS Catal. 2020, 10, 20, 11980-12010, September 22, 2020

### Summary of Invention

### Technical Problem

An object of the present invention is to provide a novel asymmetric fluorinated allylic compound with high optical purity that can be derived into various physiologically active medium molecules, and a method for producing the asymmetric fluorinated allylic compound.

### Solution to Problem

The present inventors conducted extensive research to achieve the object and succeeded in synthesizing an asymmetric fluorinated allylic compound with high optical purity that can be derived into various physiologically active medium molecules.

The present inventors conducted further research based on this finding and completed the present invention.

Specifically, the present invention encompasses the following asymmetric fluorinated allylic compounds.

### Item 1.

A compound represented by the following formula (2):

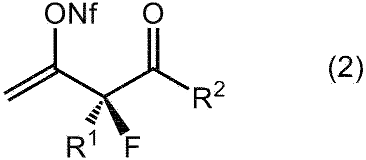

wherein
Nf represents a nonafluorobutanesulfonyl group (C₄F₉SO₂-),
R¹ represents an alkyl group, an aryl group, an aralkyl group, an alkenyl group, or an alkynyl group, and
R² represents an alkyl group, an alkoxy group, or an aryl group.

### Item 2.

A compound represented by the following formula (4): 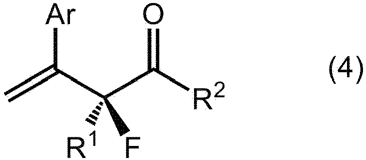 wherein
Ar represents an aryl group,
R¹ represents an alkyl group, an aryl group, an aralkyl group, an alkenyl group, or an alkynyl group, and
R² represents an alkyl group, an alkoxy group, or an aryl group.

### Item 3.

A compound represented by the following formula (5):

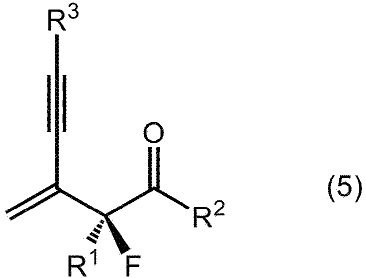

wherein
R¹ represents an alkyl group, an aryl group, an aralkyl group, an alkenyl group, or an alkynyl group,
R² represents an alkyl group, an alkoxy group, or an aryl group, and
R³ represents an alkyl group, an alkoxy group, or an aryl group.

The present invention can synthesize, for example, compound 2 (the compound represented by formula (2)), which can be converted into various compounds including special amino acids, or compound 3 (the compound represented by formula (3)), which can be converted into various compounds including special amidite acids, via compound 1 (the compound represented by formula (1)) according to a production method including a simple two-step production process.

The fluorinated allyl-containing compounds of the present invention are useful in the production of pharmaceuticals since they serve as intermediates and precursors in the synthesis of pharmaceuticals and can reduce the number of steps in the production of pharmaceuticals.

### Advantageous Effects of Invention

The present invention provides a novel asymmetric fluorinated allylic compound with high optical purity that can be derived into various physiologically active medium molecules, and a production method for the asymmetric fluorinated allylic compound.

### Description of Embodiments

The present invention is described in detail below.

Embodiments describing the present invention are intended to provide a better understanding of the concept of the invention. Unless otherwise specified, the embodiments do not limit the invention.

In the present specification, the terms "comprise" and "contain" include the concepts of comprising, consisting essentially of, and consisting of.

In the present specification, a numerical range indicated by "A to B" means "A or more and B or less."

### [1] Asymmetric Fluorinated Allylic Compound

Fluorinated allylic compounds are important raw materials in drug discovery and materials. Among fluorinated allylic compounds, asymmetric fluorinated allylic compounds having an asymmetric center are particularly important as synthetic intermediates for physiologically active substances and can be converted into medium-sized molecule precursors having various physiological activities.

The present invention can provide an asymmetric fluorinated allylic compound with high optical purity that can be derived into various physiologically active medium molecules, and a method for synthesizing the asymmetric fluorinated allylic compound.

The present invention encompasses an asymmetric fluorinated allylic compound represented by the following formula (2).

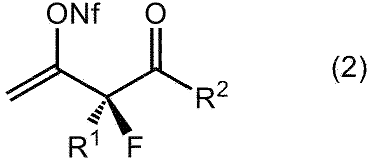

In formula (2), Nf represents a nonafluorobutanesulfonyl group (C₄F₉SO₂-). Nf is a sulfonyl protecting group.

Sulfonylating alcohols (Nf conversion) improve their desorption ability, making them active in substitution and desorption reactions. Nf has high thermal and chemical stability due to its perfluoroalkyl group, as well as extremely strong acidic properties.

In formula (2), R¹ represents an alkyl group, an aryl group, an aralkyl group, an alkenyl group, or an alkynyl group.

In formula (2), R² represents an alkyl group, an alkoxy group, or an aryl group.

The asymmetric fluorinated allylic compound represented by formula (2) (compound 2) can be converted into various compounds, including special amino acids.

The present invention encompasses an asymmetric fluorinated allylic compound represented by the following formula (4).

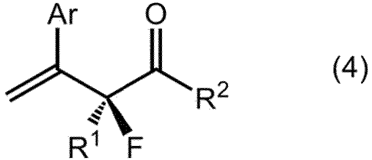

In formula (4), Ar represents an aryl group.

In formula (4), R¹ represents an alkyl group, an aryl group, an aralkyl group, an alkenyl group, or an alkynyl group.

In formula (4), R² represents an alkyl group, an alkoxy group, or an aryl group.

The present invention encompasses an asymmetric fluorinated allylic compound represented by the following formula (5).

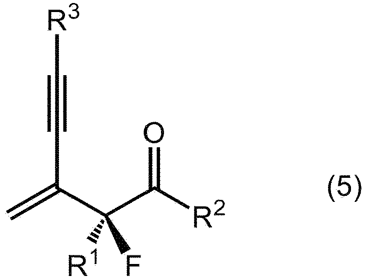

In formula (5), R¹ represents an alkyl group, an aryl group, an aralkyl group, an alkenyl group, or an alkynyl group.

In formula (5), R² represents an alkyl group, an alkoxy group, or an aryl group.

In formula (5), R³ represents an alkyl group, an alkoxy group, or an aryl group.

The asymmetric fluorinated allylic compounds represented by formulas (2), (4), and (5) of the present invention (compounds 2, 4, and 5) are asymmetric fluorinated allylic compounds with high optical purity that can be derived into various physiologically active medium molecules.

The alkyl group is preferably a linear, branched, or cyclic alkyl group. Specific examples include a C₁₋₄ linear or branched alkyl group, such as methyl, ethyl, n-propyl, isopropyl, n-butyl, isobutyl, sec-butyl, tert-butyl, and 1-ethylpropyl; a C₁₋₁₈ linear or branched alkyl group, such as n-pentyl, isopentyl, neopentyl, n-hexyl, isohexyl, 3-methylpentyl, n-heptyl, n-octyl, n-nonyl, n-decyl, n-undecyl, n-dodecyl, 5-propylnonyl, n-tridecyl, n-tetradecyl, n-pentadecyl, hexadecyl, heptadecyl, and octadecyl; and a C₃₋₈ cyclic alkyl group, such as cyclopropyl, cyclobutyl, cyclopentyl, cyclohexyl, cycloheptyl, and cyclooctyl.

The aryl group (Ar) is preferably phenyl, biphenyl, naphthyl, dihydroindenyl, 9H-fluorenyl, or the like.

The aralkyl group is preferably benzyl, phenethyl, trityl, 1-naphthylmethyl, 2-(1-naphthyl)ethyl, 2-(2-naphthyl)ethyl, or the like.

The alkenyl group is preferably ethenyl (vinyl group), 2-propenyl (allyl group), isopropenyl, butenyl, isobutenyl, tert-butenyl, pentenyl, hexenyl, heptenyl, octynyl, isooctynyl, nonenyl, or the like.

The alkynyl group is preferably ethynyl, propynyl, isopropynyl, butynyl, isobutynyl, tert-butynyl, pentynyl, hexynyl, octynyl, or nonynyl.

The alkoxy group is preferably a linear, branched, or cyclic alkoxy group. Specific examples include a C₁₋₄ linear or branched alkoxy group, such as methoxy, ethoxy, n-propoxy, isopropyloxy, n-butyloxy, isobutyloxy, sec-butyloxy, tert-butyloxy, or 1-ethylpropyloxy; a C₁₋₁₈ linear or branched alkoxy group, such as n-pentyloxy, isopentyloxy, neopentyloxy, n-hexyloxy, isohexyloxy, 3-methylpentyloxy, n-heptyloxy, n-octyloxy, n-nonyloxy, n-decyloxy, n-undecyloxy, n-dodecyloxy, 5-propylnonyloxy, n-tridecyloxy, n-tetradecyloxy, n-pentadecyloxy, hexadecyloxy, heptadecyloxy, or octadecyloxy; and a C₃₋₈ cyclic alkoxy group, such as cyclopropyloxy, cyclobutyloxy, cyclopentyloxy, cyclohexyloxy, cycloheptyloxy, or cyclooctyloxy.

The R¹, R², and R³ may each include a group having an ether bond, an ester group, an alkyl alcohol group, an amide group, or the like.

The group having an ether bond is not particularly limited.

The ester group is preferably a group with which the alkyl group described above is bonded via an ester bond. The ester group is also represented by an alkoxycarbonyl group, an alkoxycarbonylalkyl group, or a carboxyalkyl group. The ester group is preferably a methyl ester group (COOMe), an ethyl ester group (COOEt), or the like. The ester group is preferably a 3-carboxypropyl group ((CH₂)₃COOH), a 3-methyloxycarbonylpropyl group ((CH₂)₃COOMe), or the like.

The alkyl alcohol group is not particularly limited.

The amide group includes a primary amide (-NH₂), a secondary amide (-NHR¹), and a tertiary amide (-NR¹R²).

### Method for Producing Asymmetric Fluorinated Allylic Compound

The method for producing an asymmetric fluorinated allylic compound of the present invention can produce a compound represented by formula (2) (compound 2), a compound represented by formula (3) (compound 3), a compound represented by formula (4) (compound 4), and a compound represented by formula (5) (compound 5) via a compound represented by formula (1) (compound 1) .

Compound 1 is an asymmetric fluorinated allylic compound with high optical purity that can be derived into various physiologically active medium molecules.

The method for producing an asymmetric fluorinated allylic compound of the present invention can synthesize compound 2, which can be converted into various compounds including special amino acids, and compound 3, which can be converted into various compounds including special amidite acids etc. via this compound 1 by using a simple two-step production process.

The method for producing an asymmetric fluorinated allylic compound of the present invention can enable the conversion of a substituent in a double bond (compound 1 -> compound 2, compound 1 **->** compound 3).

### Method for Producing Asymmetric Fluorinated Allylic Compound Represented by Formula (2)

According to the following production method, an asymmetric fluorinated allylic compound represented by formula (2) (compound 2) (target compound) can be obtained from a compound with a structure represented by formula (1) in which the α-position of α-substituted β-diketone is fluorinated (compound 1) (substrate).

### Step of Producing Asymmetric Fluorinated Allylic Compound Represented by Formula (2)

Treating compound 1 (substrate) with a fluoride (e.g., nanoflate fluoride (NfF)) in the presence of a base (e.g., DBU) enables synthesizing a nanoflate intermediate (compound 2, target compound) with high yields.

### Substrate: Compound in Which the α-position of α-substituted β-diketone Is Fluorinated (Raw Material Compound)

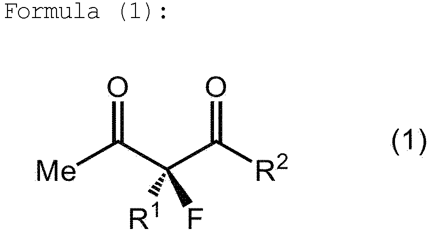

In formula (1), Me represents a methyl group.

In formula (1), R¹ represents an alkyl group, an aryl group, an aralkyl group, an alkenyl group, or an alkynyl group.

In formula (1), R² represents an alkyl group, an alkoxy group, or an aryl group.

### Organic Solvent

The organic solvent is preferably at least one organic solvent selected from the group consisting of aprotic polar solvents, halogen solvents, ether solvents, ester solvents, hydrocarbon solvents, and aromatic solvents.

The aprotic polar solvent is preferably acetone, acetonitrile, N,N-dimethylformamide (DMF), dimethylsulfoxide (DMSO), or the like.

The halogen solvent is preferably dichloromethane (DCM, CH₂Cl₂), trichloromethane (CHCl₃), or the like.

The ether solvent is preferably tetrahydrofuran (THF), dioxane, diethyl ether (Et₂O), isopropyl ether (IPE), or the like.

The ester solvent is preferably ethyl acetate (AcOEt) or the like.

The hydrocarbon solvent is preferably n-hexane or the like.

The aromatic solvent is preferably toluene or the like.

The organic solvent is more preferably a halogen solvent, such as dichloromethane or trichloromethane.

The amount of the organic solvent used is adjusted so that the concentration of the substrate (M (mol/L)) is preferably 0.01 M to 10 M, more preferably 0.02 M to 5 M, and even more preferably 0.1 M to 1 M.

### Fluoride

The fluoride is preferably a sulfonyl fluoride, such as nanoflate fluoride (perfluorobutanesulfonyl fluoride, 1,1,2,2,3,3,4,4,4-nonafluorobutane-1-sulfonyl fluoride, C₄F₉SO₂F), heptafluoropropane-2-sulfonyl fluoride ((CF₃)₂CFSO₂F)), or heptadecafluoro-n-octanesulfonyl fluoride (1,1,2,2,3,3,4,4,5,5,6,6,7,7,8,8,8-heptadecafluorooctane-1-sulfonic acid fluoride, (CF₃)(CF₂)₇SO₂F).

The amount of fluoride used is preferably 1 to 20 equivalents, more preferably 2 to 10 equivalents, and even more preferably 3 to 8 equivalents, in terms of molar ratio relative to the substrate (when the substrate is taken as 1 equivalent).

### Base

The base for use may be an inorganic base, an organic base, or the like. The base for use is preferably an organic base. The organic base for use is more preferably an organic base having strong basicity and relatively weak nucleophilicity, such as diazabicycloundecene (DBU), diazabicyclononene (DBN), or 1,4-diazabicyclo[2.2.2]octane (DABCO).

DBU has strong basicity due to the amidine moiety and is relatively weakly nucleophilic.

DBN has low nucleophilicity and belongs to the non-nucleophilic bases.

The amount of the base used is preferably 1 to 20 equivalents, more preferably 2 to 10 equivalents, and even more preferably 3 to 8 equivalents, in terms of molar ratio relative to the substrate (when the substrate is taken as 1 equivalent).

### Reaction Temperature and Reaction Time

The reaction temperature is preferably -100°C to 50°C, more preferably -100°C to 0°C, and even more preferably -100°C to -20°C.

The reaction time is preferably 1 hour to 100 hours, more preferably 2 hours to 50 hours, and even more preferably 6 hours to 48 hours.

### Target Compound: Asymmetric Fluorinated Allylic Compound

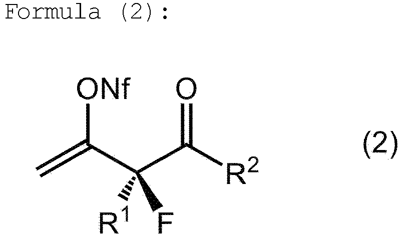

In formula (2), Nf represents a nonafluorobutanesulfonyl group (C₄F₉SO₂-).

In formula (2), R¹ represents an alkyl group, an aryl group, an aralkyl group, an alkenyl group, or an alkynyl group.

In formula (2), R² represents an alkyl group, an alkoxy group, or an aryl group.

The asymmetric fluorinated allylic compound represented by formula (2) (compound 2) is an asymmetric fluorinated allylic compound with high optical purity that can be derived into various physiologically active medium molecules. Compound 2 is a compound that can be converted into various compounds, including special amino acids.

### Method for Producing Asymmetric Fluorinated Ethynyl Group-containing Compound Represented by Formula (3)

During the production of a nanoflate intermediate (compound 2, target compound) from compound 1 (substrate) above, an asymmetric fluorinated ethynyl group-containing (HC≡C-) compound represented by formula (3) (compound 3) (target compound) can be synthesized as a by-product.

### Substrate: Compound in Which the α-position of α-substituted β-diketone Is Fluorinated (Raw Material Compound)

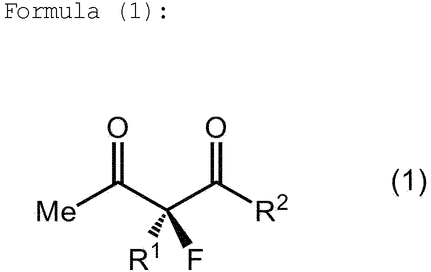

In formula (1), Me represents a methyl group.

In formula (1), R¹ represents an alkyl group, an aryl group, an aralkyl group, an alkenyl group, or an alkynyl group.

In formula (1), R² represents an alkyl group, an alkoxy group, or an aryl group.

### Target Compound: Asymmetrically Fluorinated Ethynyl Group-containing Compound

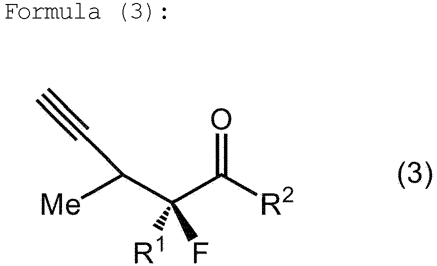

In formula (3), Me represents a methyl group.

In formula (3), R¹ represents an alkyl group, an aryl group, an aralkyl group, an alkenyl group, or an alkynyl group.

In formula (3), R² represents an alkyl group, an alkoxy group, or an aryl group.

The asymmetric fluorinated ethynyl group-containing compound represented by formula (3) (compound 3) can be converted into various compounds, including special amidite acids.

### Method for Producing Asymmetric Fluorinated Allylic Compound Represented by Formula (4)

According to the following production method, an asymmetric fluorinated allylic compound represented by formula (4) (compound 4) (target compound) can be obtained from an asymmetric fluorinated allylic compound represented by formula (2) (substrate).

### Step of Producing Asymmetric Fluorinated Allylic Compound Represented by Formula (4)

The synthesis method for use may be the Suzuki coupling (Suzuki-Miyaura coupling).

The synthesis method for use may be a chemical reaction in which an organoboron compound (e.g., ArB(OH)₂) and aryl halide (substrate compound 2) are subjected to cross-coupling by the action of a palladium catalyst (e.g., Pd(PPh₃)₄) and a nucleophilic species, such as a base (e.g., K₂CO₃), to obtain an asymmetric biaryl (biphenyl derivative) (target compound 4).

### Substrate: Asymmetric Fluorinated Allylic Compound (Raw Material Compound)

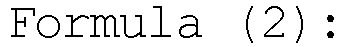

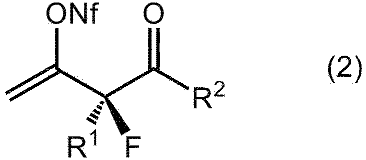

In formula (2), Nf represents a nonafluorobutanesulfonyl group (C₄F₉SO₂-).

In formula (2), R¹ represents an alkyl group, an aryl group, an aralkyl group, an alkenyl group, or an alkynyl group.

In formula (2), R² represents an alkyl group, an alkoxy group, or an aryl group.

The asymmetric fluorinated allylic compound represented by formula (2) (compound 2) can be converted into various compounds, including special amino acids.

### Organic Solvent

The organic solvent is preferably a polar solvent, such as an ether solvent.

The ether solvent is preferably tetrahydrofuran (THF), dioxane, diethyl ether (Et₂O), isopropyl ether (IPE), or the like.

The amount of the organic solvent used is adjusted so that the concentration of the substrate (M (mol/L)) is preferably 0.01 M to 10 M, more preferably 0.02 M to 5 M, and even more preferably 0.1 M to 1 M.

### Palladium Catalyst (e.g., Pd(PPh₃)₄)

The palladium catalyst for use is preferably tetrakis(triphenylphosphine)palladium (Pd(PPh₃)₄) or the like.

The amount of the palladium catalyst used is preferably 1 to 10 mol%, more preferably 2 to 8 mol%, and even more preferably 3 to 6 mol%, in terms of molar ratio as a concentration relative to the substrate (when the substrate is taken as 100 mol%).

### Bases (e.g., K₂CO₃)

The base for use is preferably potassium carbonate (K₂CO₃) or the like.

The amount of the base used is preferably 1 to 20 equivalents, more preferably 1.5 to 10 equivalents, and even more preferably 2 to 8 equivalents, in terms of molar ratio relative to the substrate (when the substrate is taken as 1 equivalent).

### Organoboron Compound (e.g., ArB(OH)₂)

The organoboron compound for use is preferably phenylboronic acid (ArB(OH)₂, Ar = phenyl group) or the like.

The amount of the organoboron compound used is preferably 0.1 to 10 equivalents, more preferably 0.5 to 8 equivalents, and even more preferably 1 to 6 equivalents, in terms of molar ratio relative to the substrate (when the substrate is taken as 1 equivalent).

### Reaction Temperature and Reaction Time

The reaction temperature is preferably 10°C to 90°C, more preferably 20°C to 80°C, and even more preferably 30°C to 70°C.

The reaction time is preferably 0.5 hours to 10 hours, more preferably 1 hours to 8 hours, and even more preferably 1.5 hours to 6 hours.

### Target Compound: Asymmetric Fluorinated Allylic Compound

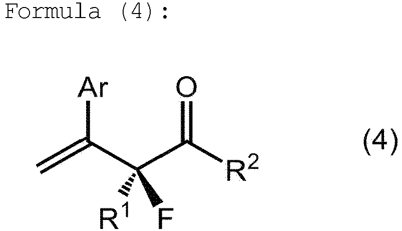

In formula (4), Ar represents an aryl group.

In formula (4), R¹ represents an alkyl group, an aryl group, an aralkyl group, an alkenyl group, or an alkynyl group.

In formula (4), R² represents an alkyl group, an alkoxy group, or an aryl group.

The asymmetric fluorinated allylic compound represented by formula (4) (compound 4) is an asymmetric fluorinated allylic compound with high optical purity that can be derived into various physiologically active medium molecules.

### Method for Producing Asymmetric Fluorinated Allylic Compound Represented by Formula (5)

According to the following production method, an asymmetric fluorinated allylic compound represented by formula (5) (target compound) can be obtained from an asymmetric fluorinated allylic compound represented by formula (2) (substrate).

### Step of Producing Asymmetric Fluorinated Allylic Compound Represented by Formula (5)

The synthesis method for use may be the Sonogashira coupling.

The synthesis method for use may be a chemical reaction in which a terminal alkyne compound (alkyne) and an aryl halide (substrate compound 2) are subjected to cross-coupling using the action of a palladium catalyst (e.g., Pd(PPh₃)₄), a copper catalyst (e.g., CuI), and a base (Et₃N, etc.) to obtain an alkynylated aryl (aromatic acetylene) (target compound 5).

### Substrate: Asymmetric Fluorinated Allylic Compound (Raw Material Compound)

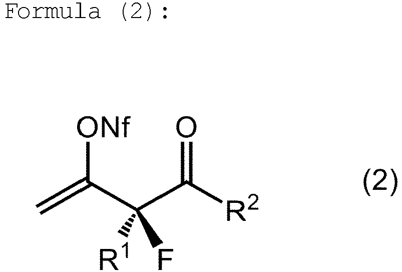

In formula (2), Nf represents a nonafluorobutanesulfonyl group (C₄F₉SO₂-).

In formula (2), R¹ represents an alkyl group, an aryl group, an aralkyl group, an alkenyl group, or an alkynyl group.

In formula (2), R² represents an alkyl group, an alkoxy group, or an aryl group.

The asymmetric fluorinated allylic compound represented by formula (2) (compound 2) can be converted into various compounds, including special amino acids.

### Organic Solvent

The organic solvent is preferably a polar solvent, such as an ether solvent.

The ether solvent is preferably tetrahydrofuran (THF), dioxane, diethyl ether (Et₂O), isopropyl ether (IPE), or the like.

The amount of the organic solvent used is adjusted so that the concentration of the substrate (M (mol/L)) is preferably 0.01 M to 10 M, more preferably 0.02 M to 5 M, and even more preferably 0.1 M to 1 M.

### Terminal Alkyne Compound (Alkyne)

The terminal alkyne compound is an alkyne compound that gives R³ (representing an alkyl group, an alkoxy group, or an aryl group) to an asymmetric fluorinated allylic compound (alkynylated aryl) represented by formula (5) (compound 5), which is a target compound.

The amount of the terminal alkyne compound used is preferably 0.1 to 10 equivalents, more preferably 0.5 to 8 equivalents, and even more preferably 1 to 4 equivalents, relative to the substrate in terms of molar ratio (when the substrate is taken as 1 equivalent).

### Copper Catalyst (e.g., CuI)

The copper catalyst for use is preferably copper(I) iodide or the like.

The amount of the copper catalyst used is preferably 1 to 40 mol%, more preferably 2 to 30 mol%, and even more preferably 5 to 25 mol%, in terms of molar ratio as a concentration relative to the substrate (when the substrate is taken as 100 mol%).

### Palladium Catalyst (e.g., Pd(PPh₃)₄)

The palladium catalyst is preferably tetrakis(triphenylphosphine)palladium (Pd(PPh₃)₄) or the like.

The amount of the palladium catalyst for use is preferably 1 to 30 mol%, more preferably 2 to 20 mol%, and even more preferably 3 to 15 mol%, in terms of molar ratio as a concentration relative to the substrate (when the substrate is taken as 100 mol%).

### Base (e.g., Et₃N)

The base for use is preferably triethylamine (Et₃N) or the like.

The amount of the base for use is preferably 1 to 20 equivalents, more preferably 1.5 to 10 equivalents, and even more preferably 2 to 8 equivalents, in terms of molar ratio relative to the substrate (when the substrate is taken as 1 equivalent).

### Reaction Temperature and Reaction Time

The reaction temperature is preferably 1°C to 50°C, more preferably 5°C to 45°C, and even more preferably 10°C to 45°C. The reaction may be performed at room temperature (1°C to 30°C).

The reaction time is preferably 0.1 hours to 10 hours, more preferably 0.2 hours to 6 hours, and even more preferably 0.3 hours to 3 hours.

### Target Compound: Asymmetric Fluorinated Allylic Compound

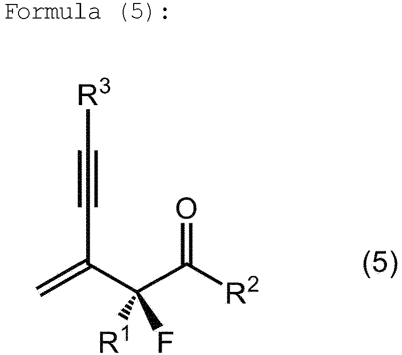

In formula (5), R¹ represents an alkyl group, an aryl group, an aralkyl group, an alkenyl group, or an alkynyl group.

In formula (5), R² represents an alkyl group, an alkoxy group, or an aryl group.

In formula (5), R³ represents an alkyl group, an alkoxy group, or an aryl group.

The asymmetric fluorinated allylic compound represented by formula (5) (compound 5) is an asymmetric fluorinated allylic compound with high optical purity that can be derived into various physiologically active medium molecules.

Embodiments of the present invention are described above; however, the present invention is not limited to these embodiments. Needless to say, the present invention can be performed in various forms without departing from the spirit and concept of the invention.

### Examples

Embodiments of the present invention are described in more detail below based on Examples.

The present invention is not limited to these Examples.

### Production of Asymmetric Fluorinated Allylic Compound Represented by Formula (2)

An asymmetric fluorinated allylic compound represented by formula (2) (compound 2) (target compound) was produced from a compound having the structure represented by formula (1) in which the α-position of α-substituted β-diketone was fluorinated (compound 1) (substrate).

### Synthesis Method

The entry compound (compound 1, substrate) was reacted with nanoflate fluoride (NfF) in the presence of a base (DBU).

### Substrate: Compound in Which the α-position of α-substituted β-diketone Is Fluorinated (Raw Material Compound)

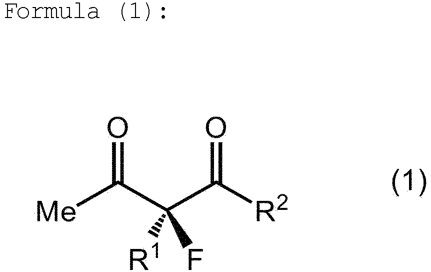

In formula (1), Me represents a methyl group.

In formula (1), R¹ represents an alkyl group, an aryl group, an aralkyl group, an alkenyl group, or an alkynyl group.

In formula (1), R² represents an alkyl group, an alkoxy group, or an aryl group.

### Target Compound: Asymmetric Fluorinated Allylic Compound

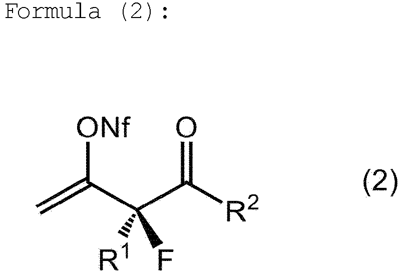

In formula (2), Nf represents a nonafluorobutanesulfonyl group (C₄F₉SO₂-).

In formula (2), R¹ represents an alkyl group, an aryl group, an aralkyl group, an alkenyl group, or an alkynyl group.

In formula (2), R² represents an alkyl group, an alkoxy group, or an aryl group.

### Production of Asymmetric Fluorinated Allylic Compound Represented by Formula (2)

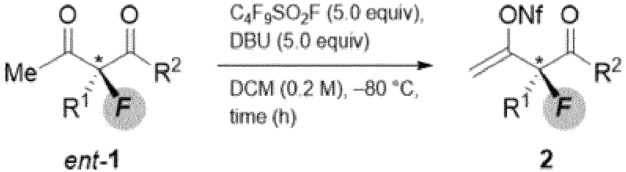

| entry | R¹ | R² | time (h)*^{b}* | yield (%)*^{c}* | *ee* (%)*^{d}* |
|---|---|---|---|---|---|
| 1 | Me | C₆H₅ | 28 | **2a:** 95 | 96 |
| 2*^{e}* | Me | 4-Me-C₆H₄ | 20 | **2b:** 94 | 94 |
| 3 | Me | 4-F-C₆H₄ | 13 | **2c:** 93 | 94 |
| 4*^{f}* | Me | OBn | 8 | **2d:** 82 | 96 |
| 5 | Bn | C₆H₅ | 28 | **2e:** 88 | 97 |

Nf: nonafluorobutanesulfonyl group (C₄F₉SO₂-)
Me: methyl group
Bn: benzyl group
C₄F₉SO₂F: fluoride; nanoflate fluoride (NfF) was used in an amount of 5.0 equivalents relative to the entry compound (substrate).
DBU: strong organic base; diazabicycloundecene was used in an amount of 5.0 equivalents relative to the entry compound (substrate).
DCM: solvent; dichloromethane (CH₂Cl₂, methylene chloride) was adjusted so that the concentration of the entry compound (substrate) was 0.2 mol/L (2 M).
Reaction temperature: the reaction was performed at -80°C.
b: The reaction time was measured by monitoring the consumption of the entry compound using TLC.
c: Isolated yield.
d: Enantioselectivity (ee(%)) was measured using HPLC equipped with a chiral stationary phase.
e: In regards to the entry-2 compound, 7.0 equivalents of NfF and 7.0 equivalents of DBU were used relative to the entry compound (substrate), and the reaction was performed at a reaction temperature of -60°C.
e: The entry-4 compound was reacted at a reaction temperature of -50°C.

According to the synthesis method described in the Examples, a nanoflate intermediate (compound 2, target compound) was synthesized with high enantioselectivity and high yield. The asymmetric fluorinated allylic compound represented by formula (2) (compound 2) is an asymmetric fluorinated allylic compound with high optical purity that can be derived into various physiologically active medium molecules. Compound 2 is a compound that can be converted into various compounds, including special amino acids.

### Production of Asymmetric Fluorinated Allylic Compound Represented by Formula (4)

An asymmetric fluorinated allylic compound represented by formula (4) (compound 4) (target compound) was produced from an asymmetric fluorinated allylic compound represented by formula (2) (compound 2) (substrate).

### Synthesis Method

The Suzuki coupling (Suzuki-Miyaura coupling) was used.

The synthesis method used was a chemical reaction in which an organoboron compound (ArB(OH)₂) and an aryl halide (substrate compound 2) were subjected to cross-coupling by the action of a palladium catalyst (e.g., Pd(PPh₃)₄) and a nucleophilic species, such as a base (e.g., K₂CO₃), to obtain an asymmetric biaryl (biphenyl derivative) (target compound 4).

### Substrate: Asymmetric Fluorinated Allylic Compound (Raw Material Compound)

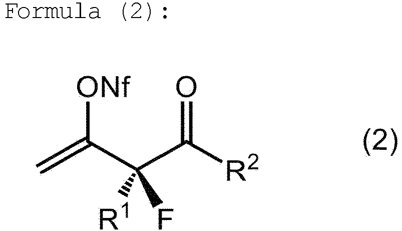

In formula (2), Nf represents a nonafluorobutanesulfonyl group (C₄F₉SO₂-).

In formula (2), R¹ represents an alkyl group, an aryl group, an aralkyl group, an alkenyl group, or an alkynyl group.

In formula (2), R² represents an alkyl group, an alkoxy group, or an aryl group.

The asymmetric fluorinated allylic compound represented by formula (2) (compound 2) can be converted into various compounds, including special amino acids.

### Target Compound: Asymmetric Fluorinated Allylic Compound

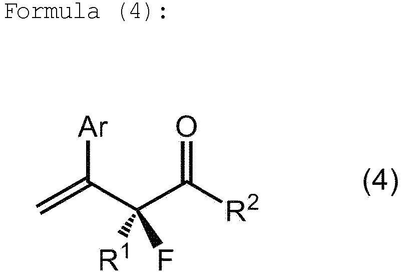

In formula (4), Ar represents an aryl group.

In formula (4), R¹ represents an alkyl group, an aryl group, an aralkyl group, an alkenyl group, or an alkynyl group.

In formula (4), R² represents an alkyl group, an alkoxy group, or an aryl group.

### Production of Asymmetric Fluorinated Allylic Compound Represented by Formula (4)

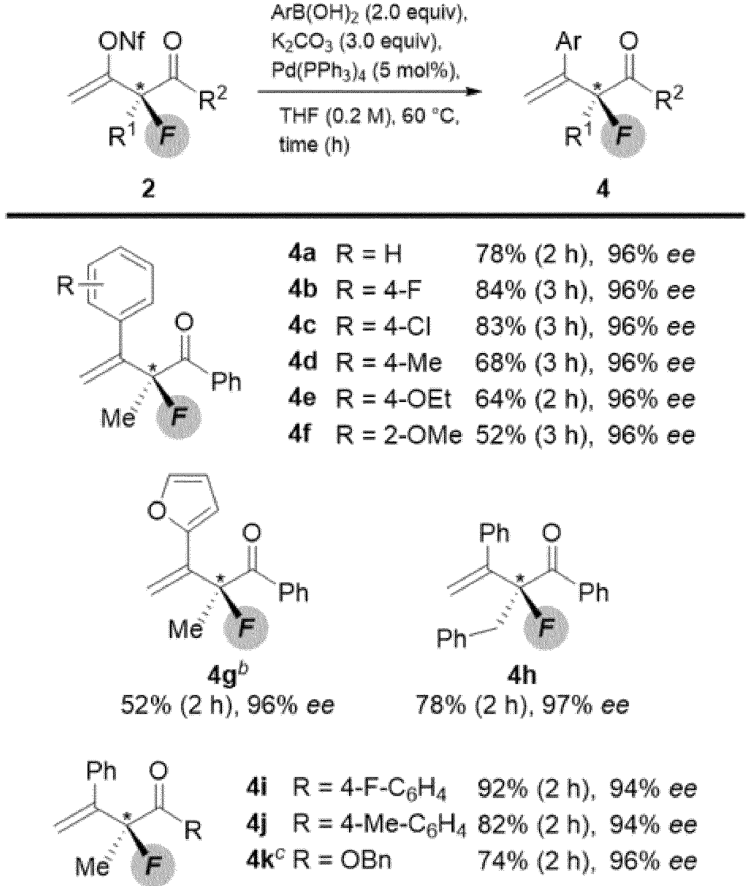

Nf: nonafluorobutanesulfonyl group (C₄F₉SO₂-)
Me: methyl group
Bn: benzyl group
Ph: phenyl group
ArB(OH)₂: Ar = phenyl group; phenylboronic acid was used in an amount of 2.0 equivalents relative to compound 2 (substrate).

K₂CO₃ (potassium carbonate) was used in an amount of 3.0 equivalents relative to compound 2 (substrate).

Pd(PPh₃)₄: palladium catalyst;
tetrakis(triphenylphosphine)palladium was used in an amount of 5 mol% relative to compound 2 (substrate).
THF: solvent; tetrahydrofuran was adjusted so that the concentration of compound 2 (substrate) was 0.2 mol/L (2M).
Reaction temperature: the reaction was performed at 60°C.

The isolated yield is shown.

The reaction time was measured by monitoring the consumption of substrate compound 2 by using TLC.

Enantioselectivity (ee(%)) was measured by using HPLC equipped with a chiral stationary phase.

b: Compound 4g was produced using 4.0 equivalents of phenylboronic acid (ArB(OH)₂) and 6.0 equivalents of K₂CO₃ relative to substrate compound 2.
c: Compound 4k was produced at a reaction temperature of 40°C.

According to the synthesis method of the Examples, an asymmetric fluorinated allylic compound represented by formula (4) (compound 4) (target compound) was synthesized with high enantioselectivity and high yield. Compound 4 is an asymmetric fluorinated allylic compound with high optical purity that can be derivatized into various physiologically active medium molecules.

### Production of Asymmetric Fluorinated Allylic Compound Represented by Formula (5)

An asymmetric fluorinated allylic compound represented by formula (5) (compound 5) (target compound) was produced from an asymmetric fluorinated allylic compound represented by formula (2) (compound 2) (substrate).

### Synthesis Method

The Sonogashira coupling was used.

The synthesis method for use was a chemical reaction in which a terminal alkyne (alkyne) and an aryl halide (substrate compound 2) were subjected to cross-coupling using the action of a palladium catalyst (e.g., Pd(PPh₃)₄), a copper catalyst (e.g., CuI), and a base (e.g., Et₃N) to obtain an alkynylated aryl (aromatic acetylene) (target compound 5).

### Substrate: Asymmetric Fluorinated Allylic Compound (Raw Material Compound)

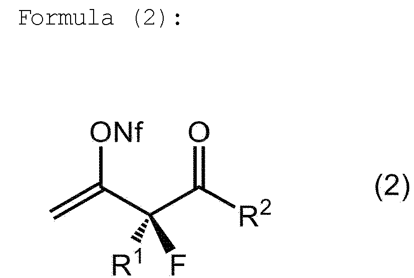

In formula (2), Nf represents a nonafluorobutanesulfonyl group (C₄F₉SO₂-).

In formula (2), R¹ represents an alkyl group, an aryl group, an aralkyl group, an alkenyl group, or an alkynyl group.

In formula (2), R² represents an alkyl group, an alkoxy group, or an aryl group.

The asymmetric fluorinated allylic compound represented by formula (2) (compound 2) can be converted into various compounds, including special amino acids.

### Target Compound: Asymmetric Fluorinated Allylic Compound

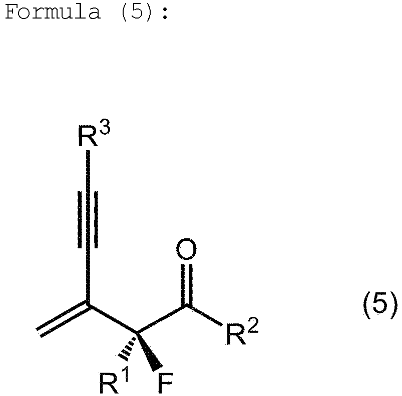

In formula (5), R¹ represents an alkyl group, an aryl group, an aralkyl group, an alkenyl group, or an alkynyl group.

In formula (5), R² represents an alkyl group, an alkoxy group, or an aryl group.

In formula (5), R³ represents an alkyl group, an alkoxy group, or an aryl group.

### Production of Asymmebic Fluorinated Allylic Compound Represented by Formula (5)

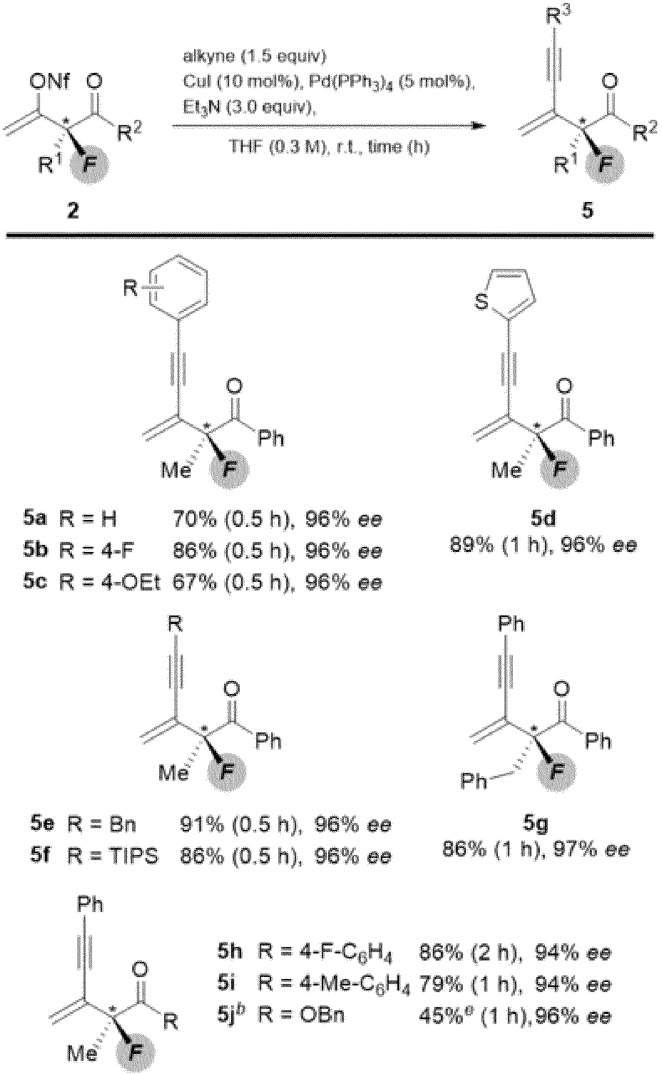

Nf: nonafluorobutanesulfonyl group (C₄F₉SO₂-)
Me: methyl group
Bn: benzyl group
Ph: phenyl group
TIPS: triisopropylsilyl group
Alkyne: an alkyne compound was used in an amount of 1.5 equivalents relative to compound 2 (substrate).
CuI: copper catalyst; copper(I) iodide was used in an amount of 10 mol% relative to compound 2 (substrate).
Pd(PPh₃)₄: palladium catalyst;
tetrakis(triphenylphosphine)palladium was used in an amount of 5 mol% relative to compound 2 (substrate).
Et₃N: base; triethylamine was used in an amount of 3.0 equivalents relative to compound 2 (substrate).
THF: solvent; tetrahydrofuran was adjusted so that the concentration of compound 2 (substrate) was 0.3 mol/L (2 M).
Reaction temperature: the reaction was performed at room temperature.

The isolated yield is shown.

The reaction time was measured by monitoring the consumption of substrate compound 2 by using TLC.

Enantioselectivity (ee(%)) was measured by using HPLC equipped with a chiral stationary phase.

b: Production of compound 5j; an alkyne compound (alkyne) was used in an amount of 3.0 equivalents relative to compound 2 (substrate). Copper(I) iodide (CuI) was used in an amount 20 mol% relative to compound 2 (substrate). A palladium catalyst (Pd(PPh₃)₄) was used in an amount 10 mol% relative to compound 2 (substrate). Triethylamine (Et₃N) was used in an amount of 6.0 equivalents relative to compound 2 (substrate). The reaction was performed at 50°C.
e: Compound 5j contained a small amount of inseparable compounds.

According to the synthesis method of the Examples, an asymmetric fluorinated allylic compound represented by formula (5) (compound 5) (target compound) was synthesized with high enantioselectivity and high yield. Compound 5 is an asymmetric fluorinated allylic compound with high optical purity that can be derivatized into various physiologically active medium molecules.

### Industrial Applicability

The present invention enables the synthesize of, for example, compound 2 (the compound represented by formula (2)), which can be converted into various compounds including special amino acids, or compound 3 (the compound represented by formula (3)), which can be converted into various compounds including special amidite acids, via compound 1 (the compound represented by formula (1)) according to a production method including a simple two-step production process.

The fluorinated allyl-containing compounds of the present invention are useful in the production of pharmaceuticals since they serve as intermediates and precursors in the synthesis of pharmaceuticals and can reduce the number of steps in the production of pharmaceuticals.

## Claims

1. A compound represented by the following formula (2): 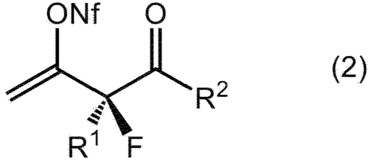 wherein
Nf represents a nonafluorobutanesulfonyl group (C₄F₉SO₂-),
R¹ represents an alkyl group, an aryl group, an aralkyl group, an alkenyl group, or an alkynyl group, and
R² represents an alkyl group, an alkoxy group, or an aryl group.

2. A compound represented by the following formula (4): 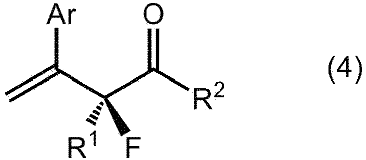 wherein
Ar represents an aryl group,
R¹ represents an alkyl group, an aryl group, an aralkyl group, an alkenyl group, or an alkynyl group, and
R² represents an alkyl group, an alkoxy group, or an aryl group.

3. A compound represented by the following formula (5): 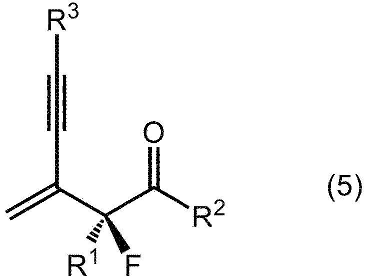 wherein
R¹ represents an alkyl group, an aryl group, an aralkyl group, an alkenyl group, or an alkynyl group,
R² represents an alkyl group, an alkoxy group, or an aryl group, and
R³ represents an alkyl group, an alkoxy group, or an aryl group.
